# EUROPÄISCHE PATENTSCHRIFT

(11) **EP 2 313 059 B1**
(45) Veröffentlichungstag und Bekanntmachung des Hinweises auf die Patenterteilung: **16.05.2012**
(21) Anmeldenummer: 09776998.8
(22) Anmeldetag: 07.07.2009
(51) Int. Cl.: A61K 8/19, A61K 8/26, A61K 8/365, A61Q 15/00, A61Q 17/00, A61K 8/04

(54) **KOSMETISCHE ZUBEREITUNGEN MIT PASSIVIERTEM SILBER**
COSMETIC PREPARATIONS WITH PASSIVATED SILVER
PRÉPARATIONS COSMÉTIQUES AVEC DE L ARGENT PASSIVÉ

(30) Priorität: 08.07.2008 DE 102008031927
(43) Veröffentlichungstag der Anmeldung: 27.04.2011
(73) Patentinhaber: Beiersdorf AG, 20253 Hamburg (DE)
(72) Erfinder: MAURER, Peter, 24536 Neumünster (DE); VOGT, Melanie, 23867 Sülfeld (DE); URBAN, Michael, 22523 Hamburg (DE); TRAUPE, Bernd, 24568 Kaltenkirchen (DE); BIEL, Stefan, 21077 Hamburg (DE); WEINERT, Katrin, 22763 Hamburg (DE); WINDISCH, Björna, 22607 Hamburg (DE)
(86) Internationale Anmeldenummer: PCT/EP2009/004895
(87) Internationale Veröffentlichungsnummer: WO 2010/003627

(56) Entgegenhaltungen:
- EP-A- 0 275 695
- WO-A-2005/041861
- WO-A-2006/029213
- WO-A-2007/096160
- DE-U1-202008 014 407
- ANONYMOUS: "Nivea Men Anti-Perspirant Silver Protect 250ml"[Online] XP002548624 Gefunden im Internet: URL:http://www.ocado.com/webshop/product/N ivea-Men-AntiPerspirant-Silver-Protect/483 03011> [gefunden am 2009-10-01]
- ANONYMOUS: "CIBA TINOSAN SDC"[Online] XP002548625 Gefunden im Internet: URL:http://www.ciba.com/ext-hpc-new2007q4p restsdc20070919.pdf?wobj=62680> [gefunden am 2009-10-01]
- ISAAC BARSHAD: 'Adsorptive and Swelling Properties of Clay-Water System' CLAYS AND CLAY MINERALS, [Online] 01 Januar 1952, Seiten 70 - 77, XP055005409 Gefunden im Internet: <URL:http://www.clays.org/journal/archive/v olume 1/1-1-70.pdf> [gefunden am 2011-08-23]

## Beschreibung

Die Erfindung beschreibt wasserfreie kosmetische oder dermatologische Zubereitungen umfassend wasserlösliche Silbercitrat-Zitronensäurekomplexe in Kombination mit Passivierungsmitteln gewählt aus der Gruppe der Schichtsilikate. Die Zubereitung ist sprühfähig als Aerosol und umfasst insbesondere ein oder mehrere Antitranspirantwirkstoffe.

Hinlänglich bekannt ist der Einsatz von Silber als Desinfektionsmittel in der Wundbehandlung, medizinischen Geräten sowie in pharmazeutischen und kosmetischen Zubereitungen.

Im Gegensatz zu wässrigen Lösungen löslicher Silbersalze (z. B. Silbernitrat oder Silberacetat) handelt es sich bei wässrigen kolloidalen Silberlösungen entweder um flüssige Dispersionen elementaren Silbers oder um flüssige Dispersionen komplexer schwerlöslicher Silberverbindungen. Die wasserdispergierbaren Ausgangsstoffe enthalten Silberionen dann nur in Spuren.

Silber bildet wie andere Edelmetalle mit Wasser als Dispersionsmittel relativ stabile kolloidale Lösungen. Für die Herstellung kolloidaler Silberlösungen gibt es unterschiedliche Verfahren. Das Silber liegt dabei im elementaren Zustand vor. In dem Dispersionskolloid werden Teilchengrößen zwischen 1 und 100 nm beobachtet, meist zwischen 10 und 50 nm.

Ein Bestreben liegt in der Bereitstellung von nicht-kolloidalem Silber.

Die EP 1128824 beschreibt nicht-kollodiale Silberverbindungen mit freien Silberionen in Kombination mit Wasser und einem thiolfreien Komplexierungsmittel, das aus Amino- und/oder Hydroxysäuren wie Lysin oder Mandelsäure, gewählt werden kann.

US 20050266081 beschreibt antimikrobielle Hydrogele enthaltend Silbersalze, Tone als Viskositätsregulatoren und Elekrolyte. Als Silbersalze sind neben den bevorzugten Silberlactaten auch Silbercitrate genannt. Als Tone werden u.a. Montmorillonit ähnliches, monoklines Tonmineral genannt. Die Hydrogele umfassen mindestens 70% Wasser.

Die erwähnten Tone dienen, wie in wässrigen Zubereitungen nicht anders zu erwarten, als Viskositätsregulatoren. Wasserfreie, bzw. bis maximal 5 Gew.% Wasser, Zubereitungen werden nicht offenbart.

WO 2006029213 A1 beschreibt antimikrobiell wirksame Silbercitratverbindungen. Diese in wässriger Zitronensäure stabilisierten Silberverbindungen sind im Handel unter TINOSAN® SDC erhältlich.

Die Herstellung dieser Verbindungen über einen elektrochemischen Prozess wird in der EP 1041879 A1 beschrieben. TINOSAN® SDC umfasst elektrolytisch erzeugte Silberionen (Ag+) und es liegt kein kolloidales Silber in der Mischung vor.

TINOSAN® SDC umfasst im Wesentlichen einen wasserlöslichen Silbercitrat-Zitronensäurekomplex der Form: 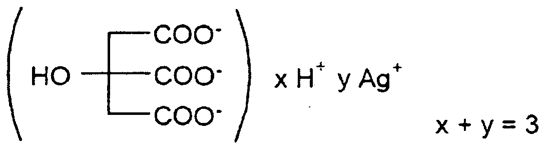 mit einem Wassergehalt von ca. 80 %, Zitronensäure ca. 20 %, Silbergehalt von ca. 2280 - 2640 ppm (ca. 0,25 %).

Das in den Druckschriften WO 2006029213 A1 und EP 1041879 A1 beschriebene Silbercitrat (TINOSAN® SDC) kann in kosmetischen, in wässriger und/oder emulsionsbasierter Zusammensetzung und mit verschiedenen Zusatzstoffen verwendet werden. Insbesondere werden Deodorantien in Kombination mit Tinosan® SDC in der WO 2006029213 A1 beschrieben.

Die in der WO 2006029213 beschriebenen Beispielzubereitungen sind vornehmlich wasserhaltig.

Zwar wird in der WO 2006029213 generell die Möglichkeit der Sprühapplikation erwähnt, jedoch werden diesbezüglich nur wasserhaltige Desinfektionssprays erwähnt, was hinsichtlich der geschilderten Probleme von wasserhaltigen Antitranspirant-Aerosole nicht verwunderlich erscheint.

Problematisch zeigt sich der Einsatz von in Wasser gelösten Silberverbindungen aufgrund von Korrosion bei metallischen Aufbewahrungsmitteln, wie beispielweise Aerosoldosen. Dieser Effekt wird verstärkt durch die Gegenwart von die Korrosion fördernden Verbindungen, wie zum Beispiel saure Aluminium- und/oder Aluminium/Zirkoniumsalze. Im Aerosol kommt es bei Wasserkontakt außerdem häufig auch zu Verstopfung der Ventile sowie bei anderen kosmetischen Applikationsformen zu Verklumpung, Instabilitäten und Inhomogenitäten.

Ein weiteres Problem bei wasserhaltigen Mischungen kann bei der Verwendung von wasserlöslich verkapseltem Parfum auftreten. In Gegenwart von Wasser können diese verkapselten Parfume quellen, verkleben und mit Auflösung reagieren.

Wünschenswert ist es demnach kosmetische oder dermatolgische Zubereitungen bereit zu stellen, die eine Darreichung von in Wasser gelösten antimikrobiell wirksamen Silberverbindungen aus eigentlich wasserfeindlicher Umgebung als Aerosol ermöglichen.

Gelöst werden die Probleme durch eine erfindungsgemäße kosmetische oder dermatologische Aerosolzubereitung gemäβ Anspruch 1.

Bevorzugt umfasst die Zubereitung zusätzlich ein oder mehreren Antitranspirantwirkstoffe.

Wasserfreie Zubereitung bedeutet erfindungsgemäß, dass neben den im Silberkomplex enthaltenem Wasser, von bis zu 90 Gew.% in Bezug auf die Gesamtkomplexmasse, lediglich zusätzliches Wasser bis zu einem Anteil von etwa 5 Gew.%, bezogen auf die Gesamtmasse der Zubereitung, enthalten sein kann. Bevorzugt bedeutet wasserfrei ein Anteil von maximal 2 Gew.%, insbesondere 0 Gew.%, bezogen auf die Gesamtmasse der Zubereitung.

Neben dem über den Silbercitrat-Zitronensäurekomplex eingeführtem Wasser enthält die Zubereitung daher maximal 5 Gew.% weiteres Wasser, bezogen auf die Gesamtmasse der Zubereitung.

Bei einem bevorzugt maximalen Anteil an Silbercitrat-Komplex von ca. 5 Gew.% und darin enthaltenem Wasser von max. 90 Gew.%, umfasst die erfindungsgemäße Zubereitung entsprechend bevorzugt maximal 4 - 5 Gew.% Wasser, welches durch den Komplex eingeführt werden kann. Zusätzlich wird maximal 5 Gew.% weiteres Wasser in der Zubereitung akzeptiert, ohne das es zu Problemen kommt, wie sie im Stand der Technik bekannt sind.

Ein maximaler Wassergehalt von 10 Gew.%, gebildet aus Wasser aus dem Komplex und Wasser aus anderen Zubereitungsbestandteilen bzw. extra zugegebenen Wasser, kann daher erfindungsgemäß in der Zubereitung insgesamt enthalten sein ohne das es zu Einbussen bei den nachfolgend geschilderten Vorteilen der erfindungsgemäßen Zubereitung kommt. Trotz diesen Anteils an möglichem Wasser wird die erfindungsgemäße Zubereitung als wasserfrei verstanden. Die Vorteile werden nachfolgend dargelegt.

Der erfindungsgemäße wasserlösliche Silbercitrat-Zitronensäurekomplex kann entsprechend aus den in der WO 2006029213 A1 und EP 1041879 A1 beschriebenen Komplexen gewählt werden. Der Offenbarungsgehalt dieser Schriften ist vollumfänglich auch Offenbarungsgehalt der vorliegenden Erfindung.

Der dort beschrieben Silberkomplex, der auch käuflich als TINOSAN® SDC erworben werden kann, ermöglicht erfindungsgemäß die Bereitstellung nicht-kolloidalen Silbers.

Die Erfindung ermöglicht den Einsatz dieses wasserhaltigen Silbercitrats mit seiner antimikrobiellen Wirkung in einer Umgebung, die bei Anwesenheit von Wasser normalerweise zu Instabilitäten und Lagerproblemen führt, wie zuvor ausgeführt. Zum Beispiel wäre der Einsatz des wässrigen Silbercitratkomplexes in metallisch verpackten Aerosolzubereitungen kritisch, da das Wasser die Metalldose oder Ventilteile, z.B. die Feder, korrodieren kann. Dieser Effekt wird verstärkt durch die Gegenwart von die Korrosion fördernden Verbindungen, wie zum Beispiel saure Aluminium- und/oder Aluminium/Zirkoniumsalze, wie sie in Antitranspirantzubereitungen (AT- zubereitungen) üblich sind.

Entsprechend findet sich kein Stand der Technik in denen das wässrige Silbercitrat in metallischen Verpackungen oder -teilen vorliegt.

Bekannt ist weiterhin, dass, sobald das Wasser dem Silbercitratkomplex entzogen wird, das Silber ausfällt und nicht die antimikrobielle Wirkung zeigen kann. Somit ist auch der einfache Entzug des Wasser keine Lösung. Erfindungsgemäß wurde auch dieses Problem nun gelöst.

Erfindungsgemäß wird das Wasser, welches durch den Silbercitratkomplex vorhanden ist, als auch eventuell zusätzliches Wasser (max. 5 Gew.%), durch das Passivierungsmittel komplexiert und gebunden.

Das über den Komplex zugeführte Wasser wird an das Passivierungsmittel gebunden und steht daher für schädigenden Einfluss nicht mehr zur Verfügung.

Die Passivierungsmittel der Schichtsilikate sind polymere kristalline Natriumdisilikate, die als multifunktionelle Gerüststoffe alternativ zu Pentanatriumtriphosphat oder in Ergänzung zu Zeolith-Builder-Systemen vornehmlich in Waschmittel-Formulierungen eingesetzt werden.

Es ist bekannt, dass Schichtsilikate als Ionenaustauscher die Härtebildner des Wassers (Ca- und Mg-Ionen) binden und als Alkalitrager, Puffer und Korrosionsschutzmittel fungieren können. Erfindungsgemäße Schichtsilikate werden gewählt aus Tonmineralien der Gruppe Montmorrillonit, Nontronit, Hectorit, Saponit, Sauconit, Beidellit, Allevardit, Illit, Halloysit, Attapulgit und/oder Sepiolit. Bevorzugt ist Disteardimonium Hectorit. Hectiorite sind M_{0,3}⁺(Mg_{2.7}Li_{0.3})[Si₄O₁₀(OH)₂], M⁺ meist = Na⁺, zu den Smektiten gehörendes, dem Montmorillonit ahnliches, monoklines Tonmineral.

Zu erwarten wäre nun ein weiteres Problem.

Aufgrund des Wasserentzugs durch die Schichtsilikat, steht das Wasser dem Silbercitrat nicht mehr zur Verfügung und die Ausfällung von Silbercitrat wäre die Folge.

Erfingdungsgemäß hat sich nun entgegen den zu erwartenden Erkenntnissen gezeigt, dass diese Passivierungsmittel das Wasser des Silbercitratkomplexes jedoch nur insoweit binden, dass es zu keinen negativen Begleiterscheinungen wie Korrosion etc. kommt, aber ohne dass es zu einer Ausfällung des gelösten Silbersalzes kommt. D.h. das Silber fällt trotz Wasserentzugs über die Bindung an das Passivierungsmittels nicht aus und steht als antimikrobielles Agens weiter zur Verfügung.

Überraschenderweise wird das so in der Formulierung passivierte Silber in Gegenwart von zusätzlichem Wasser wie Hautfeuchte oder Schweiß wieder in wassriger Lösung freigesetzt und kann dann seine antimikrobielle Aktivität wieder entfalten. D.h. wird die Zubereitung aus einer Metalldose, wo Wasser stört, ausgegeben, führt ein Wasserzusatz nach der Applikation, z.B. durch Schwitzen, dazu, dass das Silber frei wirken kann.

Dieser Effekt wird für an Schichtsilikaten passiviertes Silber insbesondere erreicht in Gegenwart von Schweiß, da die hierin enthaltenen Natriumionen das Silber aus den Bindungsstellen im Schichtsilikat verdrängen helfen.

Die Passivierung des Silbercitrats über die Passivierungsmittel Schichtsilikate führt somit zu eine Zubereitung in denen das Komplexwasser des Silbercitrates keine negativen Wirkungen entfalten kann und gleichzeitig aber zur Stabilisierung des Silbercitrats zur Verfügung steht.

Es hat sich dabei gezeigt, dass das Silber in die Schichtsilikate mit eingebunden wird. Die antimikrobielle Wirksamkeit geht aber dadurch nicht verloren, sondern wird über die Freisetzung des Silbers in Kontakt mit Wasser wieder aktiviert.

Vorteilhaft ist dieser erfindungsgemäße Wirkmechanismus beispielsweise für Deodorantien oder Antitranspirantien indem die antimikrobielle Wirkung des Silbercitrats erst durch Anschwitzen am Wirkort passiert.

Die erfindungsgemäße Kombination ist daher insbesondere für Aerosolzubereitungen vorteilhaft, da das Wasser aus dem Silbercitrat zur Aktivierung von Schichtsilikaten genutzt und damit gebunden wird. Durch diesen speziellen Prozess behält das Silbercitrat eine ausreichende Hydrathülle, um weiter in nicht-kolloidaler Form, d.h. als antimikrobiell aktiv vorzuliegen, gleichzeitig aber treten die unangenehmen Nebeneffekte nicht auf. Die antimikrobielle Wirksamkeit des Silbercitrats, wie z.B. die Deoleistung, bleibt somit erhalten, obwohl der Deowirker bzw. das ihn lösende Wasser eine enge Verbindung mit den Schichtsilikaten eingeht.

Schichtsilikate werden im Stand der Technik vor ihrer Anwendung normalerweise aktiviert, d.h. in eine quellbare Form überführt, indem man die Schichtsilikate mit einer polaren Flüssigkeit und hohen Scherkräften behandelt. Für wasserfreie Antitranspirant (AT)-Aerosole werden als polare Flüssigkeiten z.B. Propylencarbonat, Ethanol, Dipropylenglykol eingesetzt. Auf diese Aktivierung kann erfindungsgemäß verzichtet werden. Erfindungsgemäß wird eine Aktivierung nun unter Einbeziehung der wässrigen Lösung des Silbercitratkomplexes durchgeführt. Vorteilhaft lässt sich die Schichtsilikataktivierung optimieren, indem zusätzliches Parfum und die darin enthaltenen Lösemittel zugesetzt werden. Dadurch werden die Schichtsilikate aktiviert, was wiederum zu einer Viskositätserhöhung der Gesamtzubereitung führt und die Schichtsilikate und andere dispergierte Ingredienzien wie AT-Mittel weniger leicht, also langsamer sedimentieren. Das enthaltene Wasser ist komplett an das Schichtsilikat gebunden und kann nicht zu den negativen Effekten (Korrosion, Verklumpung von Parfumkapseln etc.pp.) führen. Darüber hinaus wird überraschend auch das Silber selbst in die Schichtsilikate mit eingebunden und erst bei Kontakt mit dem Schweiß auf der Haut wirksam freigesetzt.

Ein herkömmlicher Herstellprozess, bei dem man den Deowirker, einen wässrigen Silberkomplex erst nach der Aktivierung der Schichtsilikate durch intensive Scherung zugibt, führt dagegen zu den bekannten Nachteilen, da hier die Bindungsstellen der Schichtsilikate durch andere Formelbestandteile belegt sind und für die Wassermoleküle der Silbercitratlösung nicht mehr ausreichend zur Verfügung stehen.

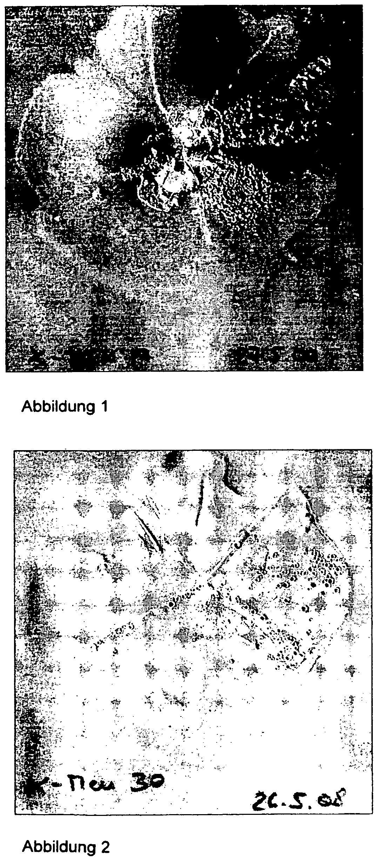

Die Bedeutung der unterschiedlichen Herstellprozesse der Aktivierung bzw. Passivierung lässt sich durch Filtrationsversuche darstellen. Im folgenden Beispiel wurde eine wasserfreie, AT-haltige Wirkstofflösung auf konventionelle Weise (Abb. 1) sowie der erfindungsgemäßen Art (Abb. 2) hergestellt und nach Lagerung über ein 200 µm Filter vakuumfiltriert.

Die Abb. 1 zeigt sehr deutlich, dass es bei konventioneller Herstellung zur Bildung von größeren, inhomogenen Agglomeraten kommt. Nachteilig daraus resultieren mögliche Verstopfungen von Aerosol-Ventilen und/oder eine ungleichmäßige Austragung des Füllgutes aus dem Packmittel. Die Austragung derartiger Agglomerate kann zudem eine negative Sensorik auf der Haut hervorrufen sowie zu vermehrten Rückständen auf Haut und Kleidung führen.

Die Abb. 2 zeigt, dass bei erfindungsgemäßer Herstellung, d.h. direkte Mischung des wässrigen Silbercitratkomplexes mit dem Passivierungsmittel ohne deren vorherige Aktivierung, die Bildung solcher Agglomerate verhindert und somit die angeführten Nachteile vermieden werden.

Die erfindungsgemäße Kombination aus Silbercitrat-Zitronensäurekomplex und Passivierungsmittel ermöglicht die Herstellung kosmetischer oder dermatologischer Zubereitungen, die erst durch externen Wasserzusatz (z.B. Schwitzen) die gewünschte antimikrobielle Wirksamkeit am Wirkort entfalten können.

Erfindungsgemäß vorteilhaft ist ebenso, dass das Wasser des Silbercitratkomlexes sowie maximal 5 Gew.% zusätzliches Wasser in der Zubereitung enthalten sein können und die Zubereitung dennoch als quasi wasserfrei wirkt, da das Wasser, wie zuvor ausgeführt, gebunden ist und keine negativen Einfluss auf die Umgebung oder weitere Bestandteile ausüben kann.

Kosmetische Antitranspirantien oder Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im allgemeinen eine mikrobielle Zersetzung von bereits gebildetem Schweiß verhindern - die Absonderung von Schweiß überhaupt verhindern sollen.

Die gewünschte Minimierung der Schweißsekretion kann durch verschiedene Mechanismen realisiert werden. Hierzu zählt traditionell der Einsatz von Adstringenzien, welche Eiweißfällungen und Gerinnungen hervorrufen und so eine Verengung oder Verschluss der Schweißdrüse bewirken.

Neuartige AT-Wirker basieren bspw. auf dem Prinzip der Anticholinergika, welche die Nervenreize, die zur Sekretionsteigerung der Schweißdrüsen führen, unterbrechen.

Ein weiteres Prinzip neuartiger AT-Wirker beruht auf der Beeinflussung von Membrantransportvorgängen in der Zelle. So hemmen spezifische Aquaporin-Inhibitoren die Proteine die Kanäle in der Zellmembran bilden, um den Durchtritt von Wasser und weiteren Molekülen zu erleichtern. Auch lonenkanal-Hemmer bewirken eine Beeinflussung von Membrantransport- bzw. Osmose-Vorgängen. Ein weiterer neuartiger AT-Wirkmechanismus lässt sich durch die Verwendung kurzkettiger, vicinaler Diole realisieren, welcher möglicherweise auf deren osmotischen Aktivität zurückzuführen ist.

So durch Anwendung eines Mittels kein Einfluss auf die Schweißsekretion ausgeübt, also keine anti-transpirante Wirkung realisiert wird, liegt erfindungsgemäß kein Antitranspirant vor.

Erfindungsgemäß sind demnach als Antitranspirantwirkstoffe solche Stoffe umfasst, die einen Einfluss auf die Schweißsekretion haben.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruches (Geruchsverbesserungsmittel). Gängige Wirkmechanismen hierzu sind antibakterielle Effekte, wie sie auch das nicht-kolloidale Silber zeigen, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Umsetzung zu wohlriechenden Stoffen umgesetzt werden.

In der WO 2006029213 A2 sind verschiedene kosmetische Zubereitungsformen, wie Shampoos oder eben auch Deodorantien erwähnt, die sich daher von den in der vorliegenden Anmeldung umfassten Antitranspirantien unterscheiden.

Zubereitungen können neben den eigentlichen schweißhemmenden Wirkstoffen (AT-Wirker) zusätzlich auch Stoffe enthalten, die den mikrobiellen Abbau des Schweißes hemmen, wie z.B. Triclosan. Triclosan wirkt gegen Gram-positive und Gram-negative Keime sowie gegen Pilze und Hefen, woraus eine desodorierende, jedoch keine antitranspirante Wirkung resultiert, da aus der Beeinflussung der bakteriellen Hautflora keine Beeinflussung der Schweißsekretion abzuleiten ist.

Insbesondere bei Antitranspirantien auf Basis von Aluminiumsalzen kann ggf. auf den weiteren Zusatz von rein desodorierend wirkenden Substanzen verzichtet werden, da diese per se ein antimikrobielles Potential aufweisen. Des Weiteren wird durch das reduzierte Wasserangebot durch die verminderte Schweißsekretion auch das bakterielle Wachstum gehemmt.

Antitranspirantwirkstoffe im Sinne der vorliegenden Anmeldung sind insbesondere zu wählen aus
1. Klassische AT-Wirker, insbesondere Metallsalze, besonders Aluminiumsalze vorzugsweise:
   - Aluminum Chlorohydrate (ACH) und activated Aluminum Chlorohydrate (ACH' oder AACH)
   - Aluminium Sesquichlorohydrate (ASCH), Aluminum Dichlorohydrate (ADCH)
   - Aluminium-Zirconium-Komplexe, optional umfassend Glycine (AZG), Aluminum zirconium tri-, tetra-, penta-, octa-chlorohydrate, Aluminum zirconium tri-, tetra-, penta-, octachorohydrex GLY
      ferner:
   - Alumium choride, -sulfate
   - Alaune (z.B. Ammonium Alum, Potassium Alum)
   - Sodium aluminum chorhydroxy lactate und/oder
2. Neuartige AT-Wirker, die Aluminiumfrei sind, wie insbesondere
   - Anticholinergika, vorzugsweise: Glycopiperolatverbindungen, wie sie in der WO 2007141289 A1 beschrieben sind und/oder Glycopyrolat,
   - Aquaporin-Inhibitoren
   - Ionen-Kanal-Hemmer, vorzugsweise NKCC1-Hemmer und/oder
   - vicinale kurzkettige Diole, wie sie in der WO 2009007089 A2 beschrieben sind.

Als Antitranspirantwirkstoff lassen sich vorteilhaft aktivierte, saure Aluminium- und/oder Aluminium/Zirkoniumsalze einarbeiten. Bei den Aluminium-Verbindungen werden vorteilhaft wasserfreie Komplexe, bei den Aluminium/Zirkonium-Verbindungen wasser- und pufferfreie Komplexe eingesetzt. Als Puffer wird hier üblicherweise Glycin verwendet.

Die nachfolgende Auflistung vorteilhaft einzusetzender Antitranspirant-Wirker soll in keiner Weise einschränkend sein:
Aluminium-Salze (der empirischen Summenformel [Al₂(OH)ₘClₙ], wobei m+n=6):
   - Aluminiumchlorhydrat [Al₂(OH)₅Cl] x H₂O Standard Al-Komplexe: Locron L, Locron LIC, Locron LIF (Clariant), Chlorhydrol (Reheis), ACH-303 (Summit), Aloxicoll L (Giulini). Aktivierte Al-Komplexe: Reach 501 (Reheis), Aloxicoll 51 L
   - Aluminiumsesquichlorhydrat [Al₂(OH)_{4,5}Cl_{1,5}] x H₂O Standard Al-Komplexe: Aloxicoll 31L (Giulini), Westchlor 186 (Westwood Chemicals) Aktivierte Al-Komplexe: Reach 301 (Reheis)
   - Aluminiumdichlorhydrat [Al₂(OH)₄Cl₂] x H₂O

Aluminium-Zirkonium-Salze:
- Aluminium/Zirkonium Trichlorhydrex Glycin [Al₄Zr(OH)₁₃Cl₃] x H₂O x Gly Standard Al/Zr-Komplexe: Rezal 33GC (Reheis), AZG-7164 (Summit)
- Aluminium/Zirkonium Tetrachlorhydrex Glycin (GLY) [Al₄Zr(OH)₁₂Cl₄] x H₂O x Gly Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
- Aluminium/Zirkonium Pentachlorhydrex Glycin [Al₈Zr(OH)₂₃Cl₅] x H₂O x Gly Standard Al/Zr-Komplexe: Rezal 67 (Reheis), Zirkonal L540, Zirkonal L530 PG (Giulini), Westchlor ZR 80B (Westwood Chemicals)
- Aluminium/Zirkonium Octachlorhydrex Glycin [Al₈Zr(OH)₂₀Cl₈] x H₂O x Gly: Westchlor ZR 82B
- Reach AZP - 908 SUF activated Aluminum Zirkonium Tetrachlorohydrex Gl
- Reach AZZ - 902 SUF activated Aluminum Zirkonium Trichlorohydrex Glyc

Ebenso vorteilhaft können aber auch Glycin-freie Aluminium/Zirkonium-Salze eingesetzt werden.

Die erfindungsgemäßen Antitranspirantien (AT) können die Bildung des Schweißes reduzieren und wirken als Schweißhemmer.

Durch die Verwendung des antimikrobiellen Silbercitratkomplexes in kosmetischen Zubereitungen kann die Bakterienflora auf der Haut reduziert werden. Dabei werden die Geruch verursachenden Mikroorganismen wirksam reduziert. Der Schweißfluß selbst wird dadurch nicht beeinflusst, im Idealfalle wird nur die mikrobielle Zersetzung des Schweißes zeitweilig gestoppt. Wird der Silber-Komplex in Antitranspirantien eingesetzt, wird der Schweißfluss durch die AT-mittel im Ergebnis dann doch beeinflusst, jedoch nicht durch den Ag-Komplex.

Die Antitranspirant-Wirkstoffe aus den zuvor geschilderten Gruppen werden in den erfindungsgemäßen Formulierungen bevorzugt in einer Menge von 1 bis 40 Gew.%, vorzugsweise von 3 bis 15 Gew. %, bezogen auf die Gesamtmasse der Zubereitung, d.h. inklusive ggf. vorhandener Treibgase, eingesetzt.

Bei Einsatz von ca. 35 Gew.% AACH (aktiviertes Aluminium Chlorohydrat) in der Wirkstofflösung (ohne Treibgas) und einem Abfüllverhältnis von etwa 15:85 (Wirkstofflösung zu Treibgas) wird ein Anteil von etwa 5,25 Gew. % AACH im Endprodukt vorhanden sein.

AT-mittel aus der Gruppen der Anticholinergika, wie beispielsweise 4-[(2-Cyclopentyl-2-hydroxyphenylacetyl)oxy]-1,1-dimethyl-piperidiniumsalze, insbesondere das 4-[(2-Cyclopentyl-2-hydroxyphenylacetyl)oxy]-1,1-dimethyl-piperidiniumbromid können zu einem Anteil von bevorzugt 0,05 bis1,0 Gew.%, vorzugsweise 0,1%-0,7%, insbesondere 0,3%-0,5 Gew.%, bezogen auf die Gesamtmasse der Zubereitung zugesetzt werden.

Auch vicinale Diole und ähnliche Wirkstoffe aus der Gruppe der osmotisch aktiven Substanzen können als AT-wirker den erfindungsgemäßen Zubereitungen zugesetzt werden. Hier dann bevorzugt zu einem Anteil von 10 bis 50 gew.%, vorzugsweise 15%-30%, insbesondere 15 -25 Gew.%, bezogen auf die Gesamtmasse der Zubereitung.

Zusätzlich ist selbstverständlich möglich weitere Antitranspirant Wirkstoffe und/oder Deodorantien zu zusetzen.

Die Passivierungsmittel, insbesondere Disteardimonium Hectorit, werden zu einem Anteil von 1 bis 10 Gew.%, insbesondere im Bereich von 2 bis 5 Gew.%, bezogen auf die Gesamtmasse der Zubereitung zugegeben,

Der Silbercitratkomplex, inklusive des Wasser des Komplexes, wird zu einem Anteil von maximal 5 Gew.%, bevorzugt 2 Gew.% zugegeben, insbesondere 0,1 bis 2 Gew.%, bezogen auf die Gesamtmasse der Wirkstoffmischung.

Die Wirkstoffmischung ist gleich der Zubereitung, als Aerosol beziehen sich die Anteilsangaben dann ggf. auf die Wirkmischung ohne Treibgas.

Die erfindungsgemäßen Zubereitungen werden vorzugsweise dargereicht in Standard Aerosol-Packmitteln. Insbesondere sind sie für die Versprühung durch Standard AT-Ventile und -Sprühköpfe geeignet, da sie Verstopfungsnachteile vermeiden helfen. Vorteil der erfindungsgemäßen Zubereitungen ist somit, dass auf aufwandige Spezial-Packmittel wie beispielsweise Puderventile verzichtet werden kann. Derartige Ventile sind aufgrund ihrer besonderen Konstruktion bzw. Geometrie weniger empfindlich für ein Verstopfen durch größere Partikel. Nachteilig an der Verwendung derartiger Pudervenille Ist aber, dass die besondere Form i.d.R. auch den Einsatz von adaptierten Sprühköpfen erfordert. Dies bedingt zusätzliche Kosten sowie eine Diversifikation des Packmittelsortiments und damit zusätzlichen logistischen Aufwand.

Diese Nachteile werden erfindungsgemäß vermieden.

Ein weiteres Problem, das erfindungsgemäß gelöst wird, ist, dass bei gleichzeitiger Verwendung von wasserlöslich verkapselten Parfums, die auf die Gegenwart von Wasser mit Quellung, Verkleben und Auflösung reagieren, auch hier diese Nachteile nicht beobachtet werden, da das in der Wirkstofflösung enthaltene Wasser an das Passivierungsmittel gebunden ist und somit keine Interaktion mit der Kapselmatrix möglich ist.

Trotz der Gegenwart von Wasser (max. 10 Gew.% (s.oben)) können so beliebige wasserlösliche Kapselmaterialien für Parfümöle erfindungsgemäß mit wassrigen Silberlösungen kombiniert werden. Bevorzugt kann somit modifizierte Starke (INCl: Sodium Starch Octenylsuccinate oder Modified Starch) als Kapselmaterial verwendet werden.

Auch hier zeigt sich erneut der erfindungsgemäß überraschende Vorteil der Zubereitung, dass obwohl Wasser enthalten ist, die Gesamtzubereitung wasserfrei wirkt.

Die Einsatzmenge an Parfümkapseln beträgt vorteilhaft im Bereich von 0,3 - 0.8 Gew. %. bezogen auf die Gesamtmasse der Zubereitung. Hinzu kommen dann bevorzugt etwa 0,8 - 1,2 Gew.% konventionelles (freies) Parfümöl. Die Parfümkapseln setzen sich bevorzugt zusammen aus etwa 40% Sodium Starch Octenylsuccinate, 10 % Mannitol und ca. 50 % Parfümöl. Bei der Auswahl des Parfümöles für die Verkapselung kann auf die freie Grundparfümierung, wie auch auf die Verkapselung von speziellen Duftrichtungen, wie beispielsweise Frische-Akkorden gesetzt werden.

Die Wirksamkeit und sensorische Akzeptanz kosmetisch dermaler Zubereitungen wird in großem Umfang vom jeweils verwendeten Vehikel beeinflusst. Die geeignete Wahl eines galenischen Trägersystems ist somit entscheidend für die spätere Anwendung und Applikation.

Bei Applikationsformen wie Aerosolen finden wasserfreie Systeme breite Verwendung. Vorteilhaft daran ist die gute Kompatibilität mit metallenen Packmitteln, wie auch die mikrobiologische und physikalische Stabilität, insbesondere in Gegenwart hoher Elektrolytkonzentrationen, wie durch AT-Mittel gegeben.

Das Nichtvorhandensein von Wasser, welches in kosmetischen Zubereitungen häufig als Lösemittel fungiert, bedingt die Verwendung alternativer Trägersysteme. Derartige Systeme können in Form von Pudern bereit gestellt werden, was allerdings nachteilig in Hinblick auf die Applizierbarkeit, die Haftung auf der Haut (und damit die Wirkdauer) sowie die kosmetische Akzeptanz sein kann.

Erfindungsgemäß umfasst die Zubereitung daher vorteilhaft eine lipophile Trägermatrix, d.h. eine kontinuierliche Phase, die wenigstens ein mit Wasser nicht mischbares flüssiges Trägeröl umfasst.

Überraschend und erfindungsgemäß bevorzugt hat die Verwendung einer lipophilen Trägermatrix gute Resultate ergeben, welche vorteilhaft ein oder mehrere Komponenten umfasst, die bei Raumtemperatur im flüssigen Zustand vorliegen, so dass bei Vorhandensein eines AT-Mittels dies fein dispergiert formuliert werden kann. Durch den Zusatz von lipophilen Verdickern, Wachsen und bei Raumtemperatur festen Komponenten lässt sich die Viskosität bzw. Konsistenz der Zubereitung entsprechend der jeweiligen Applikationsform vorteilhaft einstellen.

Neben der reinen Trägerfunktion können die lipophilen Komponenten auch eine Verminderung von augenscheinlich wahrnehmbaren weißen Rückständen durch die AT-Mittel, wie auch deren verbesserten Haftung auf der Haut bewirken. Die verbesserte Haftung auf der Haut ist auch bei zugesetzten Duftstoffen wünschenswert.

Besonders bevorzugte Trägeröle der lipophilen Trägermatrix sind zu wählen aus Cyclomethiconen, Paraffinum Liquidum, PPG-14 Butyl Ether, C12-15 Alkyl Benzoate und/oder Caprylic Capric Triglyceride.

Es ist bevorzugt eine kosmetische oder dermatologische Aerosolzubereitung bereit zu stellen, die gegebenenfalls ein Antitranspirantwirkstoff und Trägeröle gewählt aus Cyclomethiconen, Paraffinum Liquidum, PPG-14 Butyl Ether, C12-15 Alkyl Benzoate und/oder Caprylic Capric Triglyceride umfasst.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, UV-Filter, Antioxidantien, Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Nachfolgende Beispiele illustrieren die erfindungsgemäßen Zubereitungen. Die darin angeführten Anteile sind auf die Gesamtmasse der Zubereitung bzw. Wirkstofflösung bezogen.

### Beispiele:

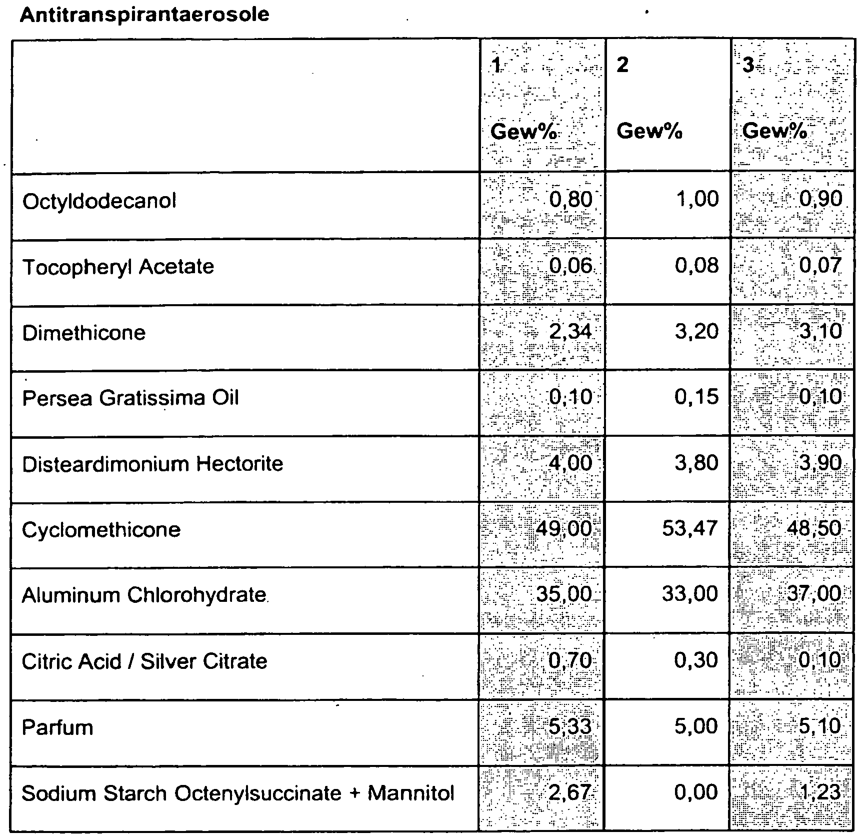

Mit Treibgas vorzugsweise Butane/Isobutane/Propane und Abfüllverhältnis 5:95 bis 30:70, vorzugsweise 10:90 bis 20:80, vorzugsweise 15:85.

## Patentansprüche

1. Kosmetische oder dermatologische Aerosolzubereitung mit einem maximalen Wassergehalt von 10 Gew.%, bezogen auf die Gesamtmasse der Zubereitung, umfassend ein oder mehrere Silbercitrat-Zitronensäurekomplexe der Form 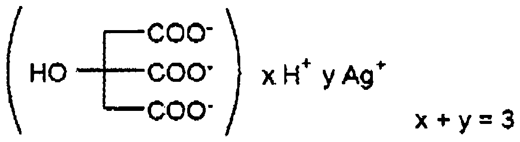 mit einem Wassergehalt von bis zu 90 Gew. %, bezogen auf die Gesamtmasse des Komplexes, in Kombination mit ein oder mehreren Passivierungsmitteln, **dadurch gekennzeichnet, dass** als Passivierungsmittel Tonmineralien gewählt werden aus der Gruppe Montmorrillonit, Nontronit, Hectorit, Saponit, Sauconit, Beidellit, Allevardit, Illit, Halloysit, Attapulgit und/oder Sepiolit und der Anteil an Passivierungsmitteln 1 bis 10 Gew.% , bezogen auf die Gesamtmasse der zubereitung, gewählt wird.

2. Zubereitung nach Anspruch 1 umfassend zusätzlich ein oder mehrere Antitranspirantwirkstoffe.

3. Zubereitung nach Anspruch 2, **dadurch gekennzeichnet, dass** als Antitranspirantwirkstoffe saure Aluminium- und/oder Aluminium/Zirkoniumsalze gewählt werden.

4. Zubereitung nach einem der vorstehenden Ansprüche umfassend verkapseltes Parfum, wobei das Kapselmaterial wasserlöslich ist.

5. Zubereitung nach Anspruch 4, **dadurch gekennzeichnet, dass** als Kapselmaterial Sodium Starch Octenylsuccinate gewählt wird.

6. Zubereitung nach Anspruch 1 umfassend Disteardimonium Hectorit als Passivierungsmittel.

7. Zubereitung nach einem der vorstehenden Ansprüche, **dadurch gekennzeichnet, dass** die Schichtsilikate nicht aktiviert eingesetzt werden.

8. Zubereitung nach einem der vorstehenden Ansprüche umfassend eine lipophile Trägermatrix.

9. Zubereitung nach Anspruch 8 **dadurch gekennzeichnet, dass** als lipophile Trägermatrix Öle gewählt werden aus Cyclomethiconen, Paraffinum Liquidum, PPG-14 Butyl Ether, C12-15 Alkyl Benzoaten und/oder Caprylic Capric Triglyceriden.

## Claims

1. Cosmetic or dermatological aerosol preparation with a maximum water content of 10% by weight, based on the total mass of the preparation, comprising one or more silver citrate-citric acid complexes of the form 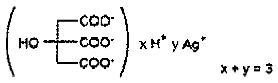 with a water content of up to 90% by weight, based on the total mass of the complex, in combination with one or more passivating agents, **characterized in that**, as passivating agent, clay minerals are selected from the group montmorillonite, nontronite, hectorite, saponite, sauconite, beidellite, allevardite, illite, halloysite, attapulgite and/or sepiolite, and the fraction of passivating agents is chosen to be 1 to 10% by weight, based on the total mass of the preparation.

2. Preparation according to Claim 1, additionally comprising one or more antiperspirant active ingredients.

3. Preparation according to Claim 2, **characterized in that** acidic aluminium salts and/or aluminium/zirconium salts are selected as antiperspirant active ingredients.

4. Preparation according to one of the preceding claims, comprising encapsulated perfume, where the capsule material is water-soluble.

5. Preparation according to Claim 4, **characterized in that** sodium starch octenylsuccinate is chosen as capsule material.

6. Preparation according to Claim 1, comprising disteardimonium hectorite as passivating agent.

7. Preparation according to one of the preceding claims, **characterized in that** the sheet silicates are used in unactivated form.

8. Preparation according to one of the preceding claims, comprising a lipophilic carrier matrix.

9. Preparation according to Claim 8, **characterized in that**, as lipophilic carrier matrix, oils are selected from cyclomethicones, paraffinum liquidum, PPG-14 butyl ether, C12-15 alkyl benzoates and/or caprylic/capric triglycerides.

## Revendications

1. Composition cosmétique ou dermatologique en aérosol, présentant une teneur maximale en eau de 10% en poids, par rapport à la masse totale de la composition, comprenant un ou plusieurs complexes citrate d'argent-acide citrique de formule 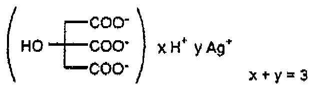 présentant une teneur en eau allant jusqu'à 90% en poids, par rapport à la masse totale du complexe, en combinaison avec un ou plusieurs agents de passivation, **caractérisée en ce qu'**on choisit, comme agent de passivation, des minéraux argileux dans le groupe formé par la montmorillonite, la nontronite, l'hectorite, la saponite, la sauconite, la beidellite, l'allevardite, l'illite, l'halloysite, l'attapulgite et/ou la sépiolite et la proportion d'agents de passivation est de 1 à 10% en poids par rapport à la masse totale de la composition.

2. Composition selon la revendication 1, comprenant en outre une ou plusieurs substances actives anti-transpirantes.

3. Composition selon la revendication 2, **caractérisée en ce que** les substances actives anti-transpirantes sont des sels d'aluminium et/ou d'aluminium/zirconium acides.

4. Composition selon l'une quelconque des revendications précédentes, comprenant un parfum encapsulé, le matériau encapsulé étant soluble dans l'eau.

5. Composition selon la revendication 4, **caractérisée en ce qu'**on choisit, comme matériau d'encapsulation, de l'octénylsuccinate sodique d'amidon (Sodium Starch Octenylsuccinate).

6. Composition selon la revendication 1, comprenant de l'hectorite de distéardimonium (Disteardimonium Hectorit) comme agent de passivation.

7. Composition selon l'une quelconque des revendications précédentes, **caractérisée en ce que** les silicates à couches sont utilisés sous forme non activée.

8. Composition selon l'une quelconque des revendications précédentes, comprenant une matrice support lipophile.

9. Composition selon la revendication 8, **caractérisée en ce qu'**on choisit, comme matrice support lipophile, des huiles parmi les cyclométhicones, la paraffine liquide (Paraffinum Liquidum), le PPG-14 butyléther, les benzoates de C12-15-alkyle et/ou les triglycérides capryliques/capriques.
